# EUROPEAN PATENT SPECIFICATION

(11) **EP 3 917 587 B1**
(45) Date of publication and mention of the grant of the patent: **03.06.2026**
(21) Application number: 20702605.5
(22) Date of filing: 28.01.2020
(51) Int. Cl.: A61L 31/02, A61L 31/10, A61L 31/14, A61L 31/16, A61K 31/337, A61K 31/436, A61P 35/00, A61P 1/18

(54) **COVERED STENT FOR LOCAL DRUG DELIVERY**
BEDECKTER STENT ZUR LOKALEN ARZNEISTOFFABGABE
ENDOPROTHÈSE VASCULAIRE COUVERTE SERVANT À L'ADMINISTRATION LOCALE DE MÉDICAMENT

(30) Priority: 30.01.2019 US 201962798498 P
(43) Date of publication of application: 08.12.2021
(73) Proprietor: BIOTRONIK AG, 8180 Bülach (CH)
(72) Inventor: BETTS, Ronald, E., La Jolla, CA 92037 (US); SAVAGE, Douglas, R., La Jolla, CA 92037 (US)
(74) Representative: WSL Patentanwälte Partnerschaft mbB
(86) International application number: PCT/EP2020/052005
(87) International publication number: WO 2020/157044

(56) References cited:
- WO-A1-2013/182503
- US-A1- 2014 086 971

## Description

### FIELD OF THE INVENTION

The present invention is directed to a drug eluting stent system for use in delivering at least one therapeutic agent to a target. Further, the present invention relates to a method for preparing the drug eluting stent system.

### BACKGROUND OF THE INVENTION

Stents are tubular devices made of plastic or metal and are used to develop or maintain patency of an animal or human vessel. Stents may be placed endoscopically or through percutaneous methods to treat the common bile duct or the main pancreatic duct to relieve obstruction. These devices effectively are employed to drain gallbladder and pancreatic fluid collections. Plastic stents represent the lower cost alternative of the devices available but they are limited in diameter to 12 French (0.156 inches) which is the allowable maximum possible that can be accommodated by a standard endoscope. For drainage of larger vessels several plastic stents or an expandable metal stent would be used. Metallic stents can be balloon expandable or constructed of materials which are self-expanding upon deployment from a delivery catheter. Either type of metallic stent can be made available with a covering made of polymer or silicone. This covering prevents ingress of tissue from the surrounding vessel lumen.

Obstruction of the bile duct or pancreatic duct can occur due to benign hyperplasia resulting from inflammation or from malignancy of the pancreas or bile duct and surrounding tissues. Its development may contribute to poor clinical outcomes including pain and delay in further treatment. Biliary obstruction correlates with decreased survival times in cancer patients. In the U.S. over 50,000 persons and over 400,000 persons worldwide are diagnosed with pancreatic cancer each year and as many as 70% of patients have some degree of biliary obstruction at the time of diagnosis. Pancreatitis affects 9 million persons worldwide. The role of biliary stents in achieving patency in obstructions or strictures of the bile duct has been well established with more recent studies showing an increased role for self-expanding metal stents. An increasing number of patients with resectable, or surgically treatable pancreatic cancers are receiving neoadjuvant chemoradiation or chemotherapy prior to surgery to increase its potential benefit. With a neoadjuvant approach surgery is delayed many months and hence there is a need for effective and durable biliary drainage, particularly driven by the fact that many chemotherapy agents require adequate liver function.

Maintaining patency of the bile duct with a stent is a requirement for further treatment of disease and to facilitate surgery at a later date. Yet review of the available literature reveals that the failure rate of stents used for treatment of malignant obstruction ranges from 19% to 46%. The predominant cause of stent failure is hyperplasia or tumor growth either into or around the implanted stent. Clogging of the stent lumen with bile salts or impacted food constitutes the secondary cause of stent failure. The addition of a covering material to the stent will reduce but not eliminate tissue ingrowth. The surface of the prosthesis may be covered entirely or it may be constructed so that defined ends of the device are exposed without covering.

Previous attempts have been made to release drug from a covered prosthesis. A membrane comprised of polymer and the drug paclitaxel was wrapped around a conventional self-expanding stent and deployed in the main bile duct of thirty-six pancreatic cancer patients in an effort to maintain patency of the duct during neoadjuvant therapy or while waiting for surgery. The duct patency duration of the patients did not significantly differ from those patients who had traditional treatment with a covered stent without drug. Several possible explanations could explain this outcome. The paclitaxel drug may not have been stable or effective in the higher pH environment present in the main bile duct. The amount of drug or the availability of the drug to surrounding tissue may have been inadequate. The physical configuration of the stent
used in the study could have been prone to blockage from food or bile contents. The intent of the present invention is to overcome both possible shortcomings of the previously studied device.

US 2014/086971 A1 discloses drug-eluting rotational spun coatings that include one or more therapeutic agents for coating a medical device, such as a stent.

WO 2013/182503 A1 discloses a drug eluting stent comprising rapamycin 40-O-cyclic hydrocarbon esters.

It is an object of the present invention to provide for a drug eluting stent system for drug delivery in the bile or pancreatic duct which is capable providing a suitable therapeutic agent in an effective dose to prevent ingress or overgrowth of hyperplasia and tumor materials.

### SUMMARY OF THE INVENTION

The present invention provides for a drug eluting stent system according to the claims.

Any references in the description to methods of treatment refer to the products of the present invention for use in a method for treatment.

The inventive drug eluting stent system comprises a coating layer, wherein the coating is composed of a porous surface covering and at least one therapeutic agent comprising an antiproliferative agent, preferably a macrocyclic triene immunosuppressive compound. The coating layer is applied to a stent being capable of radial expansion in manner that the stent is encapsulated by the coating.

The inventive drug eluting stent system for use in delivering at least one therapeutic agent to a target, preferably the bile or pancreatic duct, is as defined in claim 1.

The porous surface covering can be configured in the form of a mesh and would ideally be biocompatible with surrounding tissue and would maintain the flexibility and radial strength of original uncovered stent. At least one portion of the porous surface covering has sufficient porosity to contain enough therapeutic agent to provide therapeutic benefit over the full period of treatment. The porous surface covering is provided with an interior surface at the luminal side of the drug eluting stent system and an exterior surface at the abluminal side of the drug eluting stent system. The drug eluting stent system is provided with interior and exterior surfaces of the surface covering having different properties

In specific embodiments, the surface covering encapsulating the stent is produced of two different sheets of a porous material.

The drug eluting stent system is preferably provided with a smooth contiguous interior surface to prevent compaction of fluid and food materials and also an effective means to prevent ingress or overgrowth of hyperplasia and tumor materials. The morphology of the interior of the stent surface is of lesser porosity than the exterior surface. The drug eluting stent system delivers drug over prescribed time duration while maintaining the patency of the vessel and minimize or inhibit tissue ingrowth.

A drug which is to be administered in the bile duct or pancreatic duct is required to have a superior efficacy and stability. The drug is desired to have both anti-proliferative and antiinflammatory properties to enable suppression tumor and endothelial growth. In the living tissue the drug would inhibit benign or malignant hyperplasia in the particular vessel environment where deployed. The drug should be non-covalently bound to the porous surface covering in order to deliver a measured release of the drug at a target site. Particular consideration is given to the pH of the bile duct fluid which differs from that which is present in venous or arterial blood. Hence, the drug needs to be stable in basic pH environment. It is also desirable that the drug be lipid soluble to facilitate absorption into the surrounding tissue. It is further desirable that the drug have a wide dosing window of therapy. Specifically, the drug should exert a therapeutic effect over a range of low to high dosage levels without concern for local toxicity. It is possible that the lack of efficacy of biliary stents with embedded paclitaxel may have been due to low compatibility for one or more of the attributes stated above.

Hence, in one aspect, the present invention provides for a drug eluting stent system comprising a macrocyclic triene immunosuppressive compound which meets all the above requirements and has the following structure: 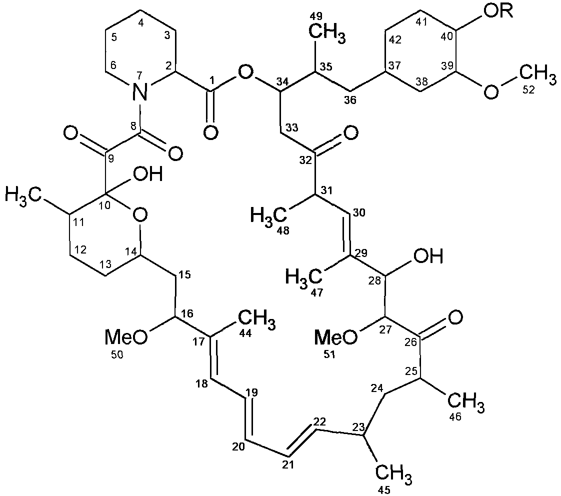 where R is C(O)-(CH₂)ₙ-X, n is 0, 1 or 2, X is a cyclic hydrocarbon having 3-9 carbons and optionally contains one or more unsaturated bonds as a coating material for medical implants, in particular for vascular stents. In a preferred embodiment of the use of the mixture, C(O)-(CH₂)ₙ₋X has one of the following structures:

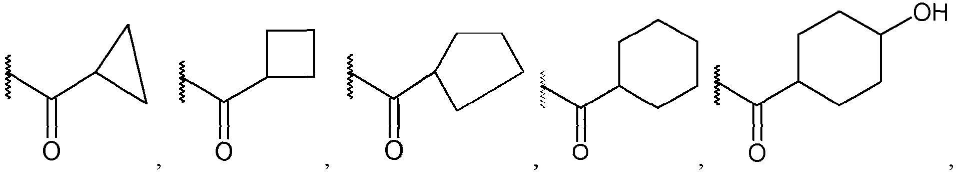

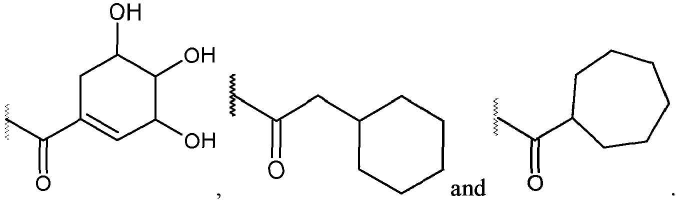

In another aspect the present invention is directed to a method for preparing a drug eluting stent system as defined in claim 10.

The therapeutic drug formulation applied to the covered stent must meet the requirements for manufacturability and efficacy. The therapeutic drug formulation comprises at least one therapeutic agent as defined herein and must be dissolved into a suitable solvent and applied as a liquid formulation through spraying, capillary application, or dip coating. The drug formulation would be maintained within porous surface covering (e.g. in the mesh lattice) as a solid mass or as captive microparticles or microspheres. It is possible that selective areas of the stent geometry could also be coated with the drug if desired. Once dried the drug must be stable at room temperature over the usable lifetime of the product. The therapeutic drug formulation comprising at least one therapeutic drug may be applied to the inner surface, the outer surface, or to both inner and outer surfaces.

### BRIEF DESCRIPTION OF THE FIGURES

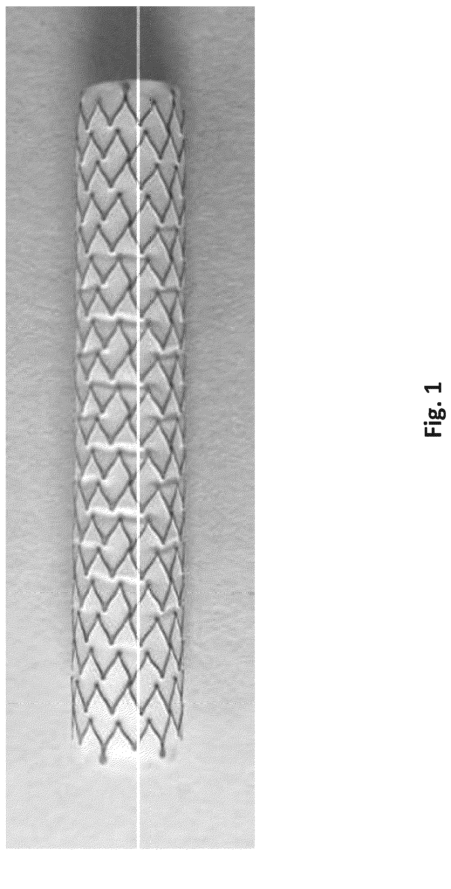

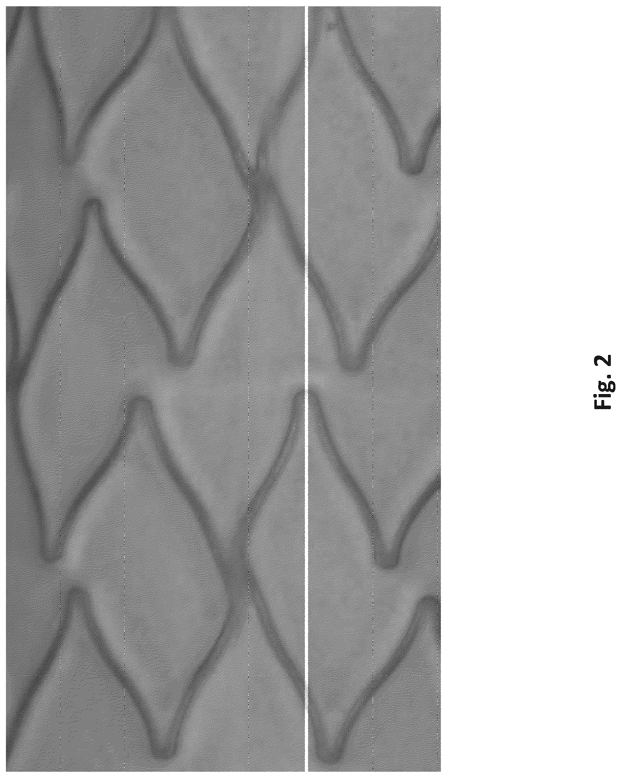

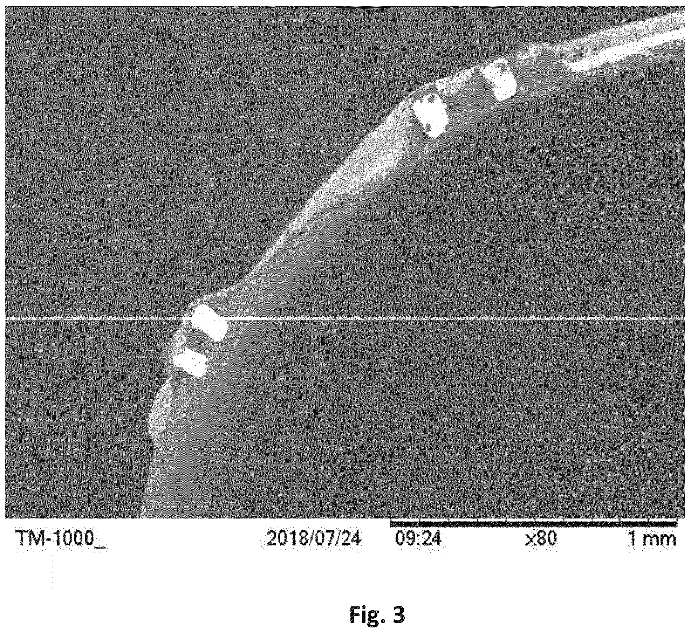

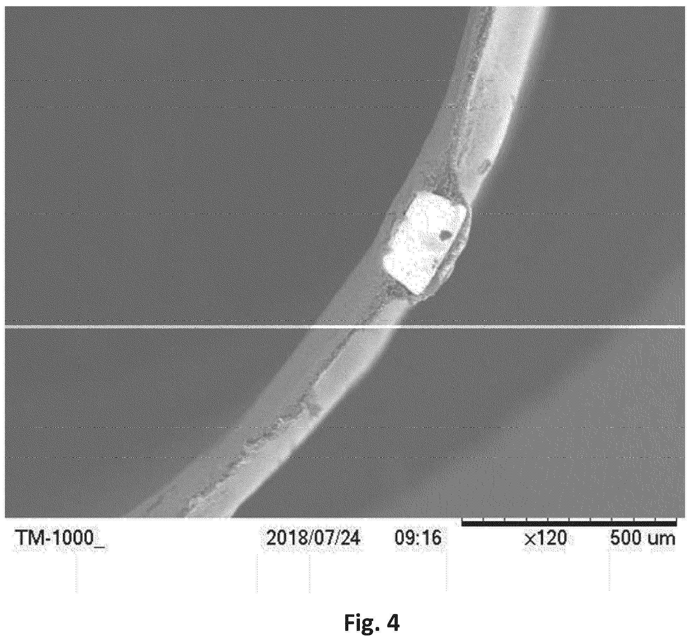

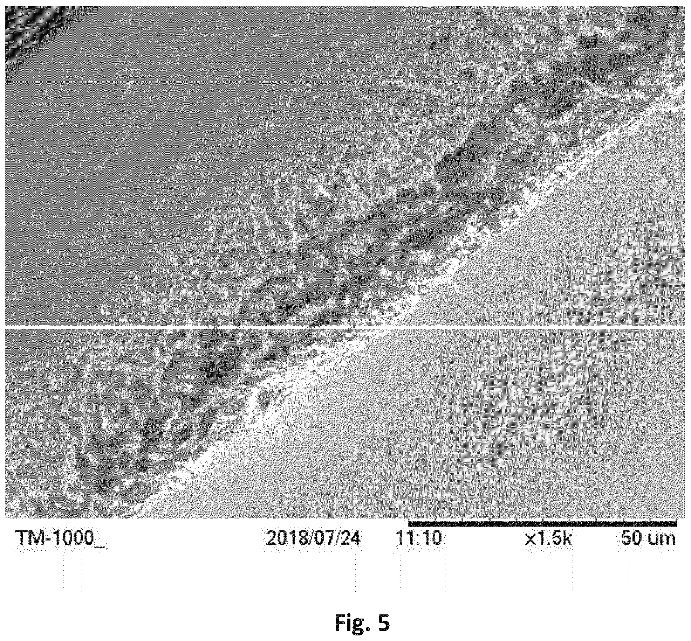

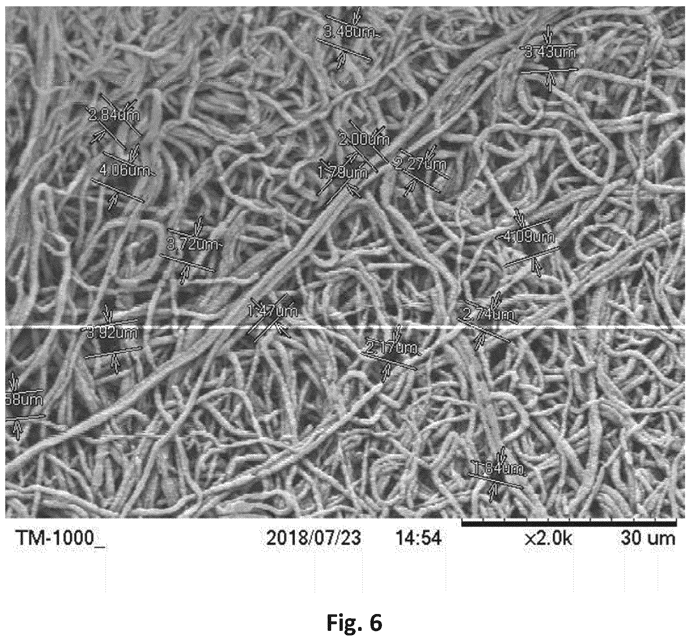

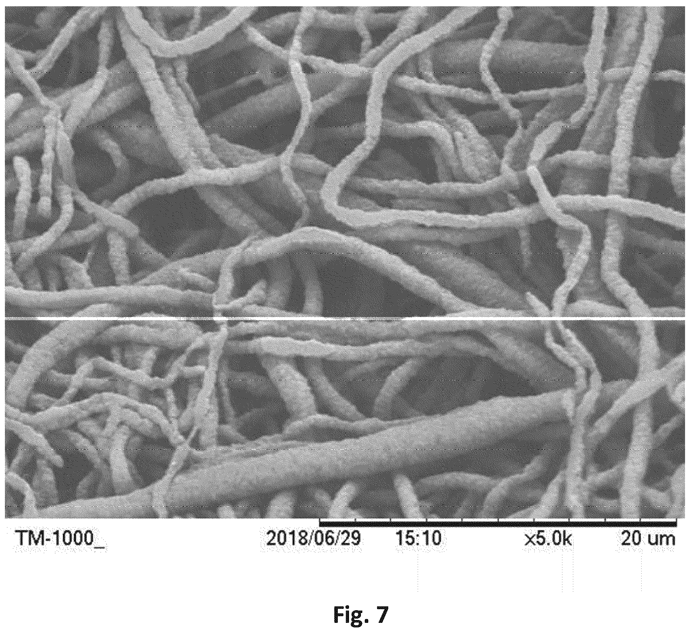

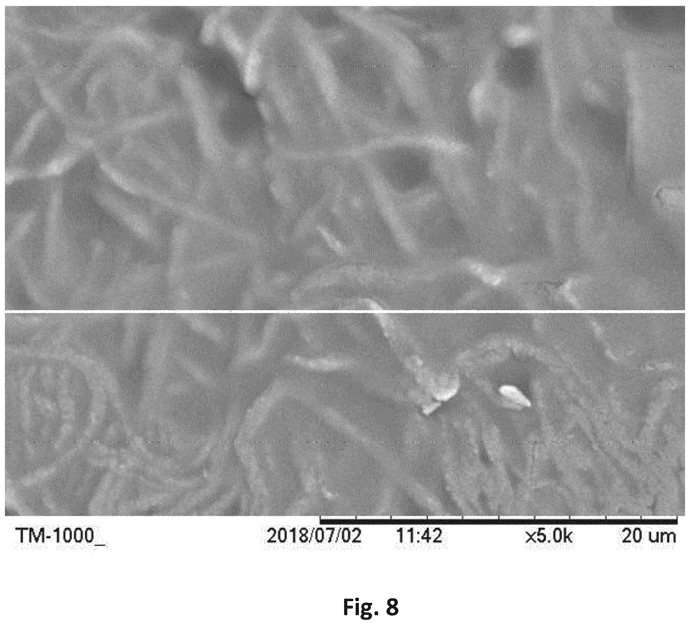

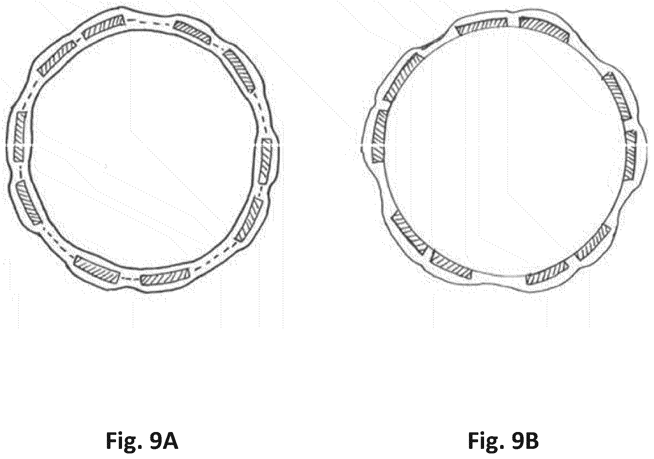

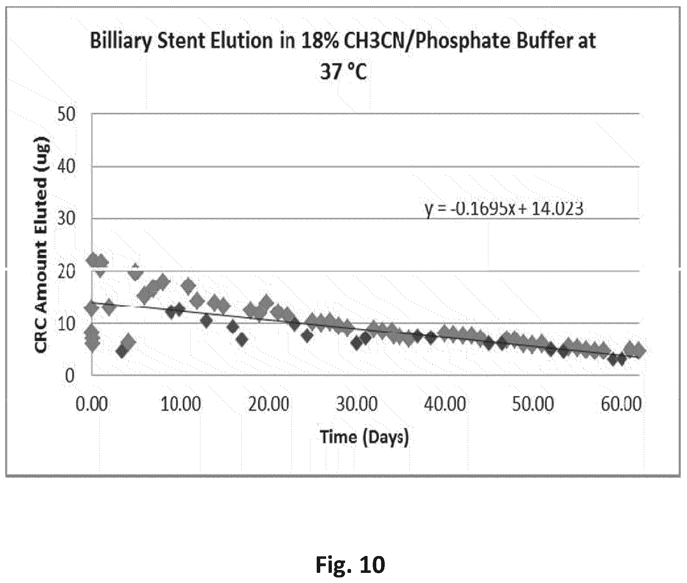

Fig. 1 is a photograph of a self-expanding stent which is encapsulated with two layers of porous electrospray PTFE film. Fig. 2 is a close-up image of the encapsulated stent showing film coverage over the strut. Fig. 3 is a scanning electron microscope image of the cross-section of the encapsulated device showing the stent struts surrounded by the porous mesh material. Fig. 4 is a scanning electron microscope image of the cross-section of the encapsulated device showing a single stent strut surrounded by the porous mesh material. Fig. 5 is a scanning electron microscope image of the porous mesh material as it appears in a cut cross-section. Fig. 6 is a scanning electron microscope image of the top view of the porous mesh with dimensions indicating the approximate pore diameter of 2.0 to 5.0 microns. Fig. 7 is a scanning electron microscope image of the top view of the porous mesh at high magnification. Fig. 8 is a scanning electron microscope image of the top view of the porous mesh with drug formulation applied into the mesh surface. Fig. 9 is a rendering of cross-sectional view of two design configurations consisting of a two-sheet encapsulation of PTFE and a single electrospray application to the stent. Fig. 10 is graph of incremental measured amount over time of CRC-015 drug released from the drug coated device in an appropriate liquid media.

### DETAILED DESCRIPTION OF THE INVENTION

### Definitions

As used herein, the term "coated" or "coating" with reference to a stent refers to coverings, compounds or substances applied to the stent surface. This would also include a drug or therapeutic compound in or on a covering, as well as any other substance that is applied to the stent surface in order to facilitate the delivery of the drug from the stent surface to a target.

As used herein, the term "elution" refers to the transfer of a drug or therapeutic agent out of the coating and is determined as the total amount of the drug or therapeutic agent excreted out of the porous surface covering as integral part of the coating.

As used herein, the term "polymer" refers to substances composed entirely of repeating structural units or monomers, connected to each other via covalent chemical bonds.

As used herein, the term "subject" refers to any animal, including humans, for which the administration of an adjuvant composition is desired. It includes mammals and non-mammals, including primates, livestock, companion animals, laboratory test animals, captive wild animals, reptiles, and fish.

As used herein, the term "encapsulated" refers to a complete surrounding of a material element with another material.

### Stent

The stent of the present invention may be produced from a suitable biocompatible metal and capable of insertion into the vasculature of an individual. The stent is comprised of a metallic base comprising a material such as stainless steel, titanium or a similar biocompatible alloy thereof.

The stent of the present invention is substantially tubular or cylindrical in design and is capable of radial expansion when located at a desired target, such as within the vasculature or proximal to a vessel wall. The stent can be configured to be self-expanding or balloon expandable.

### Coating

The present invention provides for a coating which comprises a porous surface covering that is specifically constituted to carry an extraordinarily large drug load. The porous surface covering encapsulates the stent. It is in the sense of the present application that the coating in the form of a porous surface covering expands over the interstices between stent struts as the porous surface covering formed from porous sheets which are places on the interior and/or exterior surface of the stent. The encapsulation is accomplished by applying elevated temperature and pressure to at least one sheet of porous material mounted on the stent. The porous material is in one embodiment a nonwoven fabric. The nonwoven fabric is constituted of polymeric fibers. Temperature and pressure are chosen to transfer the polymer into a semi molten state such that the fibers stick to one another upon compression. As a result of the compression a porous stent covering in the form of a thin web or mesh is produced capable of maintaining flexibility and structural integrity and further of loading and storing greater quantities of a therapeutic agent as described herein. Also, after the application of elevated temperature and pressure the porous surface covering has a sponge-like configuration capable of taking up large amounts of a drug containing formulation. The fibers of the nonwoven fabric preferably have an average diameter of 0.25 to 2.00 micrometer. Variation of the diameter of the fibers can be used to adopt a specifically desired porosity. With smaller diameters of the fibers lower degrees of porosity can be achieved. In such an embodiment the diameters of the fibers can range from 0.25 to 0.85 micrometer. According to the present inventions, the porous covering is provided with interior and exterior surfaces having different properties. Other variations in properties can reside in different materials, hydro- or lipophilicity, densities or the like as well as combinations of the aforementioned. The luminal (interior) surface is desired to be smooth in order to reduce entrapment of biliary sludge and food. Therefore, the interior surface of the porous covering exhibits a low degree of porosity below 5%, or no porosity at all. No porosity at all could be accomplished by providing a plain polymer layer e.g. in the form of a polymer film. The abluminal surface should be of sufficient porosity to store elevated therapeutic levels of drug and to efficiently elute the drug from the stent system. In that, the porous surface covering of the drug eluting stent system exhibits a porosity gradient increasing from the luminal surface to the abluminal surface of the stent system. In embodiments according to claim 1, the drug eluting stent system the porous covering has a gradient of porosity from 0% to 5% porosity at the interior (luminal) surface to 20 to 40% porosity at the exterior (abluminal) surface. Also, one embodiment of the drug eluting stent system the porous covering has a gradient of density from a high density at the interior (luminal) surface to a lower density at the exterior (abluminal) surface. The highest density of the porous covering is that of the plain polymeric material used for fibers without exhibiting any porosity at all. It is in the sense of the invention described herein that the interior surface side may exhibit no porosity at all simultaneously requiring that the other side exhibits porosity to be a porous surface covering. In that at least one surface exhibits porosity, namely the external (abluminal) surface. It shall be understood that "surface side" is directed to the surface sides of the stent having an interior, luminal side and an exterior, abluminal side. Having a surface covering from one surface side to the other necessarily leads to an encapsulation of the stent since both surfaces of the stent are covered. The thickness of the entire porous surface covering preferably ranges between 20 to 100 micrometers. In this regime the porous surface covering together with the stent is flexible and yet strong enough to resist the external pressure from tissue ingrowth.

The amount of the at least one therapeutic agent applied to the drug eluting stent system is between 0.5 µg/mm² to 5.0 µg/mm² of surface area, preferably from about 1.0 µg/mm²to 3.0µg/mm² of surface area, depending on stent size. In some cases the drug load of the at least one therapeutic agent per unit area of the stent can be from about 4.0 µg/mm² to 5.0 µg/mm² of surface area. Note that this is the plain area without taking the area of the pores into account. Hence, the at least one therapeutic agent as defined herein can be present in the porous surface covering in a concentration of at least 0.05 µg/mm², preferably of at least 0.5 µg/mm², more preferably of at least 1.0 µg/mm², and most preferably of at least 3.0 µg/mm² of surface area. According to the present invention, the amount of the at least one therapeutic agent is between 0.5 µg/mm² to 5.0 µg/mm² of surface area, preferably between 4.0 µg/mm² and 5.0 µg/mm² and is applied only to the abluminal side to the drug eluting stent system. Hence, only the abluminal surface of the porous covering is exhibiting these drug loads, whereas the luminal surface of the porous stent covering is free of the at least one therapeutic agent or exhibits only a drug load of 0.05µg/mm² to 1.0 µg/mm².

In another aspect, the therapeutic agent is preferably applied to the porous surface covering by use of a formulation comprising at least 2.0% by weight of the at least one therapeutic agent dissolved into a suitable solvent. For preparation of the formulation a solvents are preferred that are nontoxic, capable of dissolving the therapeutic agent and have a vapor pressure low enough to facilitate easy drying. Examples for solvents that fulfill at least one of these requirements are acetone, acetonitrile, tetrahydrofuran, chloroform, dichloromethane, and ethanol.

In a preferred embodiment the porous surface covering is a nonwoven fabric. The nonwoven fabric is preferably produced by way of electrospinning. During electrospinning, a polymer solution, preferably a solution comprising a nonwoven polymer as listed above, is delivered through a metal nozzle. Between the metal nozzle and the basic structure, a high voltage is applied. The basic structure has a different potential than the metal nozzle. By feeding the polymer solution and due to the voltage difference between the nozzle and the basic structure, a filament is applied onto a surface as a nonwoven fabric. The fibers of the nonwoven fabric are composed of a biocompatible polymer and preferably of a biostable biocompatible polymer. In one embodiment of the invention the electrospun fibers are composed of a polymer selected from the group comprising or consisting of include polytetrafluoroethylene, fluorinated ethylene propylene, Dacron, polyethylene terephthalate, polyurethanes, polycarbonate, polypropylene, Pebax, polyethylene and biological polymers such as collagen, fibrin, and elastin. In a further preferred embodiment the electrospun fibers are composed of polytetrafluoroethylene or polyurethane. Further, for encapsulating the stent in the porous covering, two different sheets of non-woven fabric can be applied to the luminal side of the stent and the abluminal side of the stent, respectively. In a preferred embodiment the interior surface of the porous covering is produced of polytetrafluoroethylene with low, preferably below 5%, or no porosity. Such an interior surface of the porous covering could be made of a polytetrafluoroethylene sheet as a material, preferably with a smaller fiber diameter as 1 µm, adds additional strength to the covering, which prevents ingress of hyperplasia and tumor materials. Also, additional to the encapsulation and the application of a therapeutic agent, at least one of the porous and preferably non-woven sheets used for encapsulation of the stent can carry a sealing agent to reduce or eliminate porosity at one surface, preferably the luminal surface, of the porous covering. Suitable sealing agents include but are not limited to silicone, silane compounds, polyurethanes, polysulfones, thermoplastic elastomers, pvc and other vinyl materials, fatty alcohols including docosanol, polysorbate 80 and other surfactants, and flexible, biocompatible organic polymers such as polycaprolactone.

The porous surface covering encapsulating the stent is preferably configured to have a mesh pore diameter ranging from one micron to five microns. Determination of the mesh pore diameter may done under scanning electron microscope inspection. In one aspect of the invention the porous surface covering has a porosity of within the range of 20% to 40% of the surface area and preferably of 30%. As stated above porous surface covering encapsulating the stent may have a gradient of porosity, preferably increasing in porosity from the luminal surface to the abluminal surface of the porous surface covering.

### Therapeutic agents

The at least one therapeutic agent is a macrocyclic triene immunosuppressive compound having the following structure: 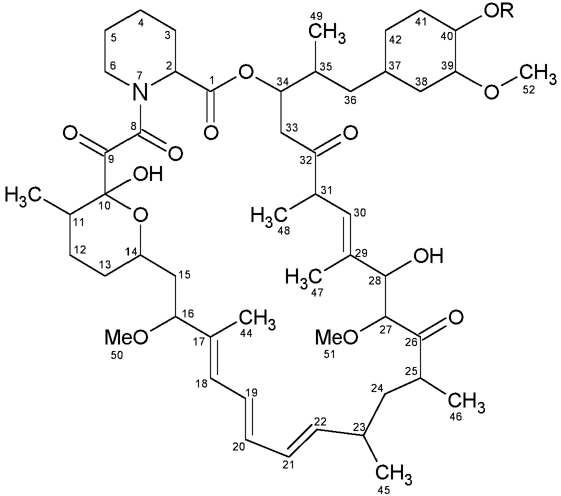 where R is C(O)-(CH₂)ₙ-X, n is 0, 1 or 2, X is a cyclic hydrocarbon having 3-9 carbons and optionally contains one or more unsaturated bonds. In a most preferred embodiment, C(O)-(CH₂)ₙ-X has one of the following structures:

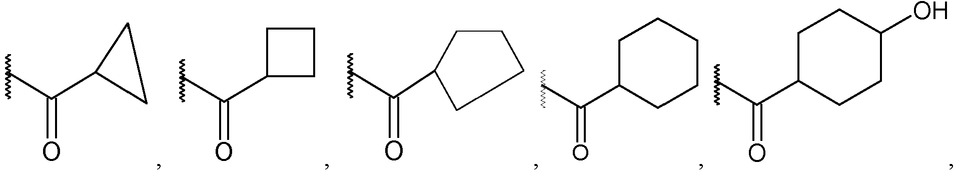

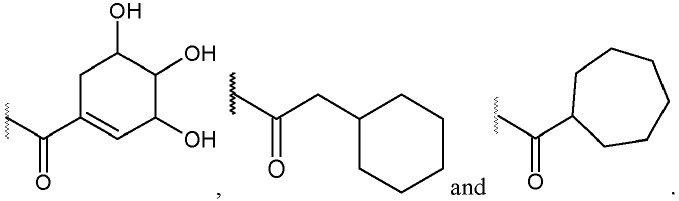

Specifically, the macrocyclic triene immunosuppressive compound of the present invention has more than one embodiment and may be described as comprising at least one of the following species from Table 1:

| Main structure | R is C(O)-(CH₂)ₙ-X having one of the following structures | Species |
|---|---|---|
| 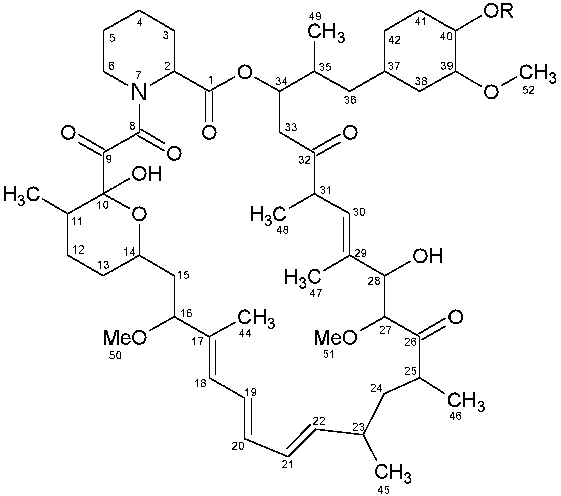 | 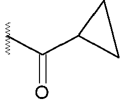 | CRC-015a |
| | 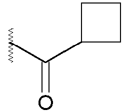 | CRC-015b |
| | 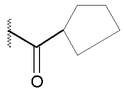 | CRC-015c |
| | 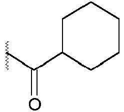 | CRC-015d |
| | 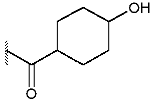 | CRC-015e |
| | 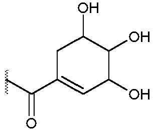 | CRC-015f |
| | 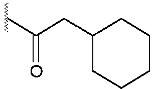 | CRC-015g |
| | 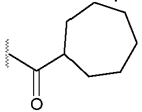 | CRC-015h |

CRC-015 is a term meant to encompass a genus and used to refer to each of the following species from Table 3: CRC-015a, CRC-015b, CRC-015c, CRC-015d, CRC-015e, CRC-015f, CRC-015g and CRC-015h.

A combination of several drugs may be applied to the stent system surface or stent system surfaces. Among the macrocyclic triene family of compounds besides the CRC-15 compounds are the drugs everolimus, zotarolimus, Biolimus, sirolimus, and Novolimus. Other drugs used to systemically treat pancreatic adenocarcinoma may be applied locally through the use of the implantable stent graft. These drugs could include gemcitibane, paclitaxel, nab-paclitaxel, docetaxel 5-fluorouracil (FU), flurouracil, irinotecan, oxaliplatin, cic-platinen, and leucovorin.

Further, it is recognized that drug combinations can have increase therapeutic effect. The CRC-015 compounds disclosed herein can be combined with cancer drugs below in conjunction with the covered stent of the present invention. These cancer drugs can be selected from the group comprising or consisting of Capecitabine, Erlotinib, Fluorouracil (5-FU), Gemcitabine, Irinotecan, Leucovorin, Paclitaxel, Nab-paclitaxel, Irinotecan and Oxaliplatin.

In one embodiment of the drug eluting stent system the surface covering has different therapeutic agents loaded to the luminal surface and the abluminal surface. One both surfaces different combinations of drugs can be loaded. In one embodiment on the abluminal surface at least one or the only therapeutic agent is a macrocyclic triene immunosuppressive compound having the following structure: 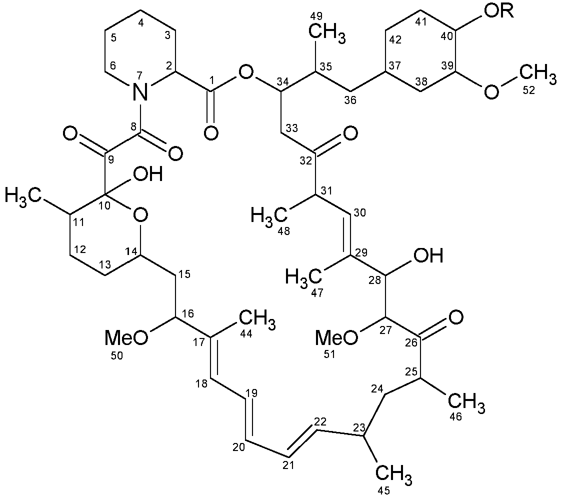 where R is C(O)-(CH₂)ₙ-X, n is 0, 1 or 2, X is a cyclic hydrocarbon having 3-9 carbons and optionally contains one or more unsaturated bonds. In a most preferred embodiment, C(O)-(CH₂)ₙ-X has one of the following structures:

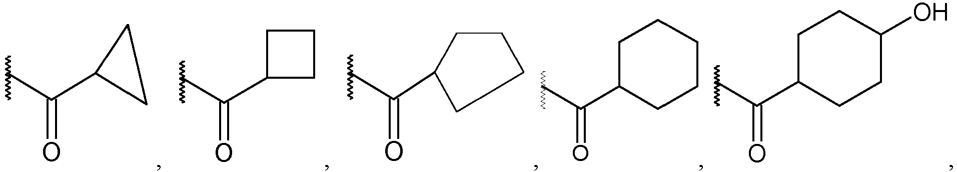

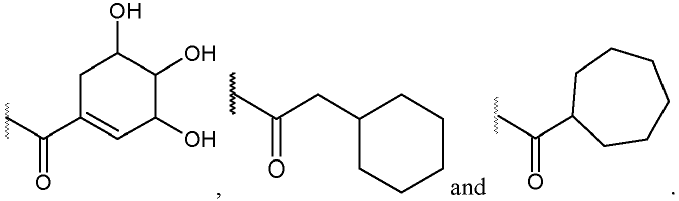

In a further embodiment the drug eluting stent system has loaded at least one of the CRC-15 compounds as defined herein one the abluminal surface and a suitable anti-coagulant on the luminal surface such as heparin. Antibiotics and other drugs or antimicrobial materials which inhibit bacterial film formation may also be applied to the luminal surface.

The at least one therapeutic agent of the coating of the drug eluting stent system as suggested herein may be accompanied by at least one suitable excipient material. Suitable excipient materials may have an effect to prolong the presence of the drug in tissue and are selected from the group comprising or consisting of polyester polymers such as polylactic acid (PLA) and/or polyglycolic acid (PGA), fatty alcohols, fatty acids, lipids, steroids, polymers, waxes, and oils.

### Elution

The drug eluting stent of the present invention further provides for a measured elution rate based on the formulation and the nature of the therapeutic agent applied to the porous surface covering and the porosity of the latter. The coating provides for a stable elution rate that is calculated based on the disassociation of the drug from the covering once the stent system is deployed. It is an advantage of the present invention that the porous surface covering is capable of storing higher drug doses that are prerequisite to cancer therapy.

In one aspect the present invention is therefore directed to a method of treating (i) bile duct obstruction or (ii) pancreatic duct obstruction or (iii) pancreatic cancer by placing a drug eluting stent system as described herein into the bile duct or the pancreatic duct.

### Method

One aspect of the invention is directed to a method for preparing drug eluting stent system as defined in claim 10. Further, step (b) may be conduct in such a manner that the sheet is mounted on the luminal and abluminal surface of a stent. Also, the method as suggested herein can be carried out such that steps (a) and (b) are conducted simultaneously by applying the sheet of porous material directly on the surface of the stent. Step (a) is preferably carried out by electrospray technique or other means, especially electro-spinning technique onto a flat surface. Respective sheets are then cut to size before application to the preferably metallic bare stent. In the embodiments according to claim 10, step (a) is conducted in such a way that two different sheets of porous material, preferably of electrospun materials are provided. Step (b) is then correspondingly carried out that one of the two sheets, of the same or of a different material, is mounted on the luminal side of a stent, whereas the second sheet of porous material is mounted on the abluminal side of the stent. Also, when a porosity gradient shall be provided for the drug eluting stent system as suggested herein more than one sheet having a different degree of porosity may be provided on each side of the stent in order to provide a more diverse gradient. In particular, it is preferred that the porosity increases from the luminal surface to the abluminal surface of the stent system. In particular for providing the interior surface of the porous covering smooth and with low or no porosity step (a) can be carried out in that at least one sheet of porous material is provided and a second sheet with less or no porosity is provided. In this embodiment the sheet of porous material is mounted on the abluminal surface of the stent, whereas the sheet of less or no porosity is mounted on the luminal surface of the stent. The resulting encapsulation of the stent after carrying out step (c) would then be accomplished between one porous material on the abluminal surface and one nonporous material on the luminal surface. Further, when a porous material on the abluminal surface is combined with a material of less porosity on the luminal surface, step (c) can be carried out in such a way that the few and/or tight pores at the luminal surface are closed by using elevated temperatures, in particular by slightly melting the material at luminal surface. Additionally, step (f) may be incorporated in the method as suggested herein comprising applying at least one sealing agent to the luminal surface of the porous surface covering.

### Examples

### Stent preparation

Example A: In one configuration the invention includes an expandable prosthesis (stent) with a base metal structure comprised of an alloy of nickel titanium which is then encapsulated with a biocompatible porous mesh. The prosthesis self-expands at human or animal body temperature to its full diameter without the aid of a deployment balloon. The mesh pore diameter under scanning electron microscope inspection measures from one micron to five microns. The tubular prosthesis is captured between two sheets of porous PFTE or polyurethane material which have been previously cast by electrospray technique or other means onto a flat surface and are then cut to size before application to the metallic bare stent. A coating comprised of the antiproliferative drug CRC-015 is dissolved into acetone and applied to the stent using a precision syringe applicator which determines the amount of drug applied. The porous mesh covering is capable of storing higher drug doses that are prerequisite to cancer therapy. The coated device is then dried at room temperature for 24 hours. When placed into an appropriate liquid elution media the drug will partition from the mesh surface into the surrounding media. An example of the encapsulated device measuring 40mm in length and 7mm in diameter was coated with 4.5mg of the drug CRC-015 dissolved in acetone solvent. After drying, the coated device was placed into a glass container with 10mL of 18% acetonitrile in phosphate buffer (w/w). The container was then placed into a Heidoph model Promax 1020 shaker table with a controlled temperature of 37°C and was shaken at a frequency of 200 strokes per minute. Samples of the liquid media were assayed for drug content. The composite curve of drug elution from the stent over time yields a consistent value of drug shown to release from the stent for a period of at least 60 days.

Example B: In another configuration a stent comprised of cobalt chromium metal mesh was covered with a polyurethane mesh directly applied using electrospray technology. After drying the covered stent is ready for drug application. A 120kHz Micromist nozzle from Sonotek Inc. is used to deposit a coating of between 10 and 35 microns depth of drug formulation containing CRC-015 is onto the outer mesh surface. After drying for 24 hours at room temperature the drug coated prosthesis is mounted onto a balloon catheter using a crimping device. In human use when the balloon is located at the site of a stricture or stenosis and inflated the device will deploy to its final diameter and the drug contained in the mesh covering may elute into surrounding tissue.

Example C: A polymer free solution of CRC-015 was prepared by dissolving the drug in acetone to prepare a solution of 30 mg/mL CRC-015. Next, paclitaxel was added to the solution and dissolved resulting in a final paclitaxel concentration of 10 mg/mL. This drug combined solution was used to coat covered stents by methods disclosed to prepare a therapeutic combination device capable of inhibiting two different metabolic processes of cancer. It is recognized that two drugs can be applied separately to the covered stents resulting with different drugs in different areas or together.

Example D: A polymer containing solution of CRC-015 was prepared by dissolving CRC-015 in acetone to prepare a solution of 20 mg/mL CRC-015. PLA was added to the solution and dissolved resulting in a final PLA concentration of 10 mg/mL. This drug polymer combined solution was used to coat covered stents by methods disclosed above. The addition of polymers can be utilized to further modify drug elution rates from the covered stent of this invention.

### Trial A

A study was conducted to evaluate the anti stenotic properties of CRC-015 on a covered self expanding stent as suggested herein implanted in the Superficial Femoral Artery (SFA) and the Profundus Artery in farm swine for 28 days. Two farm swine (species *sus scrofa, ~* 3 month of age) were sedated, intubated and placed on isoflurane for general anesthesia. All animals were pre-treated with aspirin (80mg) and Plavix^{™} for two days prior to their scheduled surgery and continued daily until their follow up procedure. Two covered stents with a dose of 2.0µg/mm² CRC-015 having a diameter of 6.0 and 7.0 mm in diameter and a length of 40 mm were deployed in the peripheral SFA and Profundus vessels, for one hind limb. The contralateral femoral artery was used for catheter and guide access. The stent graft material was 2x2 Bioweb Electrospun PTFE and there was no excipient. The electronspun graft material was pressurized at elevated temperature to encapsulate the stent as suggested herein. The delivery system was an over the wire balloon and requires a 0.035" wire via a dual lumen shaft. The shaft includes a hydrophilic surface coating. The overall length of the delivery system catheter was 150 cm. At necropsy, animals were heparinized then sodium pentobarbital were used for euthanasia. The vascular system was accessed and a sheath inserted into the arterial system. Normal saline was infused into the arterial system until the venous effluent starts to run clear from the pre-treated vessels then the treated arteries were excised. These vessels were adequately labeled, and fixed in 10% neutral formalin and delivered to a pathologist for tissue processing. In one animal the SFA appeared to be occluded while the profundus was widely patent. In the second animal both SFA and profundus were widely patent.

### Trial B

The primary purpose of this study was to evaluate the feasibility of deploying a CRC-015 DCB (drug coated balloon) and a CRC-015 coated biliary stent graft in biliary ducts of domestic swine. Two farm swines (species *sus scrofa,* at least 3 month of age) were chosen, because they offer a similar scale as human and thus the instrumentation used in human studies could be used. The animals were euthanized 27 days after successful implantation. Endpoints included duct patency and inflammatory response at 28 days. The CRC-015 DCB and CRC 015 stent graft delivery system as suggested herein was inserted through a direct needle puncture in the common bile duct near the duodenum. Puncture sites were sutured after stent deployment and delivery system retrieval. The DCB was deployed in the proximal portion of the bile duct (near the gallbladder) and the stent graft was deployed in the distal portion of the bile duct (far from the gallbladder). The Test Articles (TA) used in this study were: 6 mm x 40 mm CRC-015 DCB, a 0.035 guide wire (4 µg/mm²) and a 6 mm x 30 mm CRC-015 biliary stent graft system (4 µg/mm²) as well as 7 mm x 30 mm CRC-015 biliary stent graft system (4 µg/mm²).

After sacrificing the animals the facility veterinarian concluded that all animals were in good clinical condition and experienced a normal weight gain over the study period (from treatment to final procedures). The clinical pathology showed some iatrogenic excursions from normal ranges as commonly seen in any surgical animal model due to the blood losses, anesthesia, injectable drugs, surgery and other manipulations (eg. reticulocytosis, increase of AST (Aspartate Aminotransferase) or CK (Creatine Kinase)). No abnormal increases of Bilirubin (Total, Direct or Indirect) were reported at sacrifice suggesting that the TA do not interfere in the normal flux of the biliary duct. The two animals had some excursions pointing to an inflammatory status. One animal also had excursions in platelet count and globulins, but mainly an increased fibrinogen at sacrifice in contrast to a normal level pre-implantation. The role played for the TA, as the possible etiology or a contributor for those changes, remains elusive.

The Study Pathologist concluded that necropsy results revealed liver adhesions that were interpreted as secondary to the implantation procedures. Histopathologically, the tissue reaction to the CRC-015 Stent Graft was characterized by minimal or mild subacute inflammation in the mucosa, muscularis and/or serosa, and minimal or mild necrosis of the mucosa and minimal or mild neovascularization. These changes were considered to be related to the bile duct implantation of the stent graft. Multifocal hemorrhages, most probably related to the harvesting of the bile duct, were seen in the muscularis and/or serosa of both animals. Epithelialization of the luminal aspect of the CRC-015 Stent Graft was not observed. Fibrin infiltration, fibrosis, and mineralization were not seen in any of the stent-grafted and the proximal non-stented bile duct sections. In conclusion, the necropsy results revealed liver adhesions that were interpreted as secondary to the implantation procedures. Histopathological evaluation revealed presence of minimal or mild inflammation, neovascularization and mucosal necrosis.

## Claims

1. A drug eluting stent system for use in delivering at least one therapeutic agent to a target, preferably the bile or pancreatic duct, comprising:
(a) a stent capable of radial expansion; and
(b) a coating, wherein the coating is composed of a porous surface covering and at least one therapeutic agent, wherein the porous surface covering has a porosity of within the range of 20% to 40% of the surface area at the abluminal surface and of less than 5% of the surface area at the luminal surface and wherein the at least one therapeutic agent is an antiproliferative agent and wherein the stent is encapsulated by the coating, wherein the amount of the at least one therapeutic agent is between 0.5 and 5.0 µg/mm² of surface area and is applied only to the abluminal side to the drug eluting stent system.

2. The drug eluting stent system of claim 1, wherein the at least one therapeutic agent is a macrocyclic triene immunosuppressive compound.

3. The drug eluting stent system of claim 1, wherein the at least one therapeutic agent has the following structure: 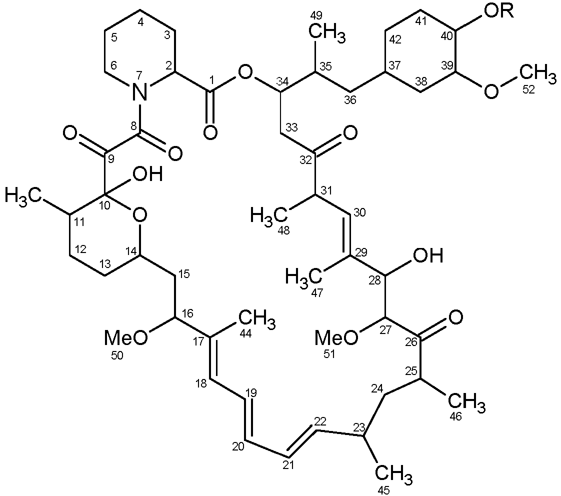 where R is C(O)-(CH₂)ₙ-X, n is 0, 1 or 2, X is a cyclic hydrocarbon having 3-9 carbons and optionally contains one or more unsaturated bonds..

4. The drug eluting stent system of claim 3, further wherein C(O)-(CH₂)ₙ-X has one of the following structures:

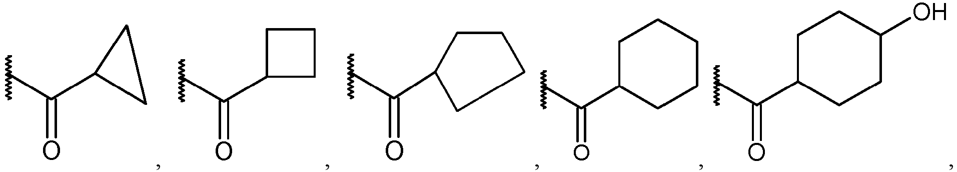

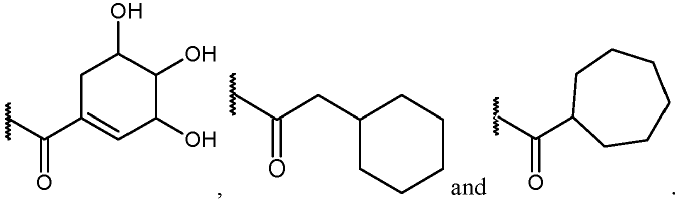

5. The drug eluting stent system of claim 1, wherein the porous surface covering is a nonwoven fabric.

6. The drug eluting stent system of claim 5, wherein the nonwoven fabric is composed of electrospun fibers.

7. The drug eluting stent system of claim 6, wherein the electrospun fibers are composed of a polymer selected from the group comprising or consisting of include polytetrafluoroethylene, fluorinated ethylene propylene, Dacron, polyethylene terephthalate, polyurethanes, polycarbonate, polypropylene, Pebax, polyethylene and biological polymers such as collagen, fibrin, and elastin.

8. The drug eluting stent system of claim 1, wherein the porous surface covering has a gradient of porosityfrom 0% porosity at the luminal surface to 20 to 40% porosity at the abluminal surface.

9. The drug eluting stent system of claim 1, wherein the amount of the at least one therapeutic agent is between 4.0 µg/mm² and 5.0 µg/mm² of surface area and is applied only to the abluminal side to the drug eluting stent system.

10. A method for preparing a drug eluting stent system comprising the steps of
- (a) providing two different sheets of a porous material,
- (b) mounting one of the two sheets on the luminal of the stent, whereas the second sheet of porous material is mounted on the abluminal side of the stent, such that the porosity increases from the luminal surface to the abluminal surface of the stent system in that the sheet on the luminal surface of the stent has less than the sheet of porous material being mounted on the abluminal surface of the stent or no porosity,
- (c) applying elevated temperature and pressure to encapsulated the stent in the porous material thereby providing a porous surface covering on the stent,
- (d) applying a solution of a therapeutic agent to the porous surface covering, and
- (e) drying the porous surface covering applied with the therapeutic agent solution, wherein in step (c) the temperature is in the range of 55°C to 90°C and the pressure is in the range of 0.13MPa to 1.25MPa.

## Patentansprüche

1. Wirkstoff freisetzendes Stentsystem zur Zuführung wenigstens eines therapeutischen Wirkstoffs zu einem Ziel, vorzugsweise den Gallengang oder den Pankreasgang, umfassend:
(a) einen radial expandierbaren Stent und
(b) eine Beschichtung, wobei die Beschichtung aus einer porösen Oberflächenschicht und wenigstens einem therapeutischen Wirkstoff besteht, wobei die poröse Oberflächenschicht eine Porosität im Bereich von 20 % bis 40 % der Oberfläche an der abluminalen Seite und eine Porosität im Bereich von weniger als 5 % der Oberfläche an der luminalen Seite aufweist und wobei der wenigstens eine therapeutische Wirkstoff ein antiproliferativer Wirkstoff ist und wobei der Stent von der Beschichtung umhüllt ist, wobei die Menge des wenigstens einen therapeutischen Wirkstoffs zwischen 0,5 und 5,0 µg/mm² der Oberfläche liegt und dieser nur auf der abluminalen Seite des Wirkstoff freisetzenden Stentsystems aufgebracht wird.

2. Wirkstoff freisetzendes Stentsystem nach Anspruch 1, wobei das wenigstens eine therapeutische Mittel eine makrozyklische, immunsuppressive Trienverbindung ist.

3. Wirkstoff freisetzendes Stentsystem nach Anspruch 1, wobei das wenigstens eine therapeutische Mittel die folgende Struktur aufweist: 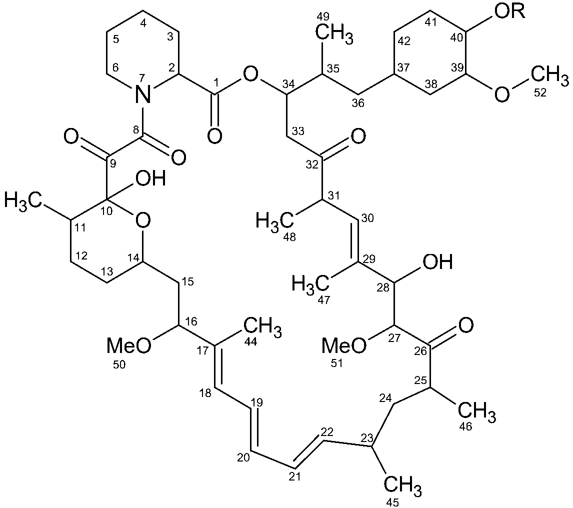 wobei R = C(O)-(CH₂)ₙ-X ist, n = 0, 1 or 2 ist, X ein zyklischer Kohlenwasserstoff mit 3-9 Kohlenstoffen ist und optional eine oder mehrere ungesättigte Bindungen aufweist.

4. Wirkstoff freisetzendes Stentsystem nach Anspruch 3, wobei C(O)-(CH₂)ₙ-X eine der folgenden Strukturen aufweist:

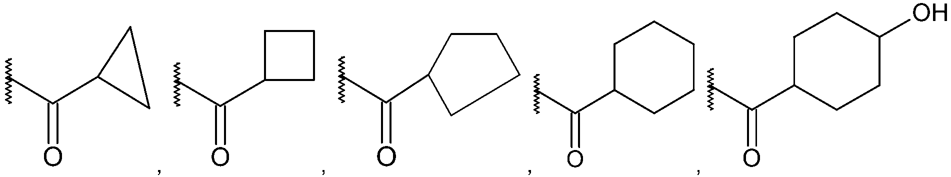

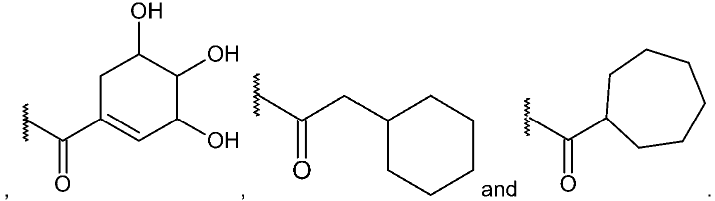

5. Wirkstoff freisetzendes Stentsystem nach Anspruch 1, wobei die poröse Oberflächenbeschichtung ein Vliesstoff ist.

6. Wirkstoff freisetzendes Stentsystem nach Anspruch 5, wobei der Vliesstoff aus elektrogesponnenen Fasern besteht.

7. Wirkstoff freisetzendes Stentsystem nach Anspruch 6, wobei die elektrogesponnenen Fasern aus einem Polymer bestehen, das aus der Gruppe ausgewählt ist, die Polytetrafluorethylen, fluoriertes Ethylen-Propylen, Dacron, Polyethylenterephthalat, Polyurethane, Polycarbonat, Polypropylen, Pebax, Polyethylen und biologische Polymere wie Kollagen, Fibrin und Elastin umfasst oder daraus besteht.

8. Wirkstoff freisetzendes Stentsystem nach Anspruch 1, wobei die poröse Oberflächenbeschichtung einen Porositätsgradienten von 0 % Porosität an der luminalen Oberfläche bis zu 20 bis 40 % Porosität an der abluminalen Oberfläche aufweist.

9. Wirkstoff freisetzendes Stentsystem nach Anspruch 1, wobei die Menge des wenigstens einen therapeutischen Wirkstoffs zwischen 4,0 µg/mm² und 5,0 µg/mm² der Oberfläche beträgt und nur auf die abluminale Seite des Wirkstoff freisetzenden Stentsystems aufgebracht ist.

10. Verfahren zur Herstellung eines Wirkstoff freisetzenden Stentsystems, umfassend die folgenden Schritte:
- (a) Bereitstellen von zwei unterschiedlichen Schichten aus einem porösen Material,
- (b) Anbringen einer der beiden Schichten auf der luminalen Seite des Stents, während die zweite Schicht aus porösem Material auf der abluminalen Seite des Stents angebracht wird, derart, dass die Porosität von der luminalen Oberfläche zur abluminalen Oberfläche des Stentsystems zunimmt, indem die Schicht auf der luminalen Oberfläche des Stents eine geringere Porosität aufweist als die auf der abluminalen Oberfläche des Stents angebrachte Schicht aus porösem Material oder gar keine Porosität,
- (c) Anwenden erhöhter Temperatur und erhöhten Drucks, um den Stent mit dem porösen Material zu umhüllen und dadurch eine poröse Oberflächenschicht auf dem Stent zu erzeugen,
- (d) Aufbringen einer Lösung eines therapeutischen Wirkstoffs auf die poröse Oberflächenschicht und
- (e) Trocknen der mit der therapeutischen Wirkstofflösung beschichteten porösen Oberflächenschicht, wobei in Schritt (c) die Temperatur im Bereich von 55 °C bis 90 °C und der Druck im Bereich von 0,13 MPa bis 1,25 MPa liegt.

## Revendications

1. Système de stent à élution de médicament destiné à être utilisé pour administrer au moins un agent thérapeutique à une cible, de préférence le canal biliaire ou le canal pancréatique, comprenant:
(a) un stent apte à une expansion radiale; et
(b) un revêtement, le revêtement étant composé d'un recouvrement de surface poreux et d'au moins un agent thérapeutique, le recouvrement de surface poreux présentant une porosité comprise entre 20 % et 40 % de la superficie au niveau de la surface abluminale et inférieure à 5 % de la superficie au niveau de la surface luminale, et ledit au moins un agent thérapeutique étant un agent antiprolifératif, le stent étant encapsulé par le revêtement, la quantité dudit au moins un agent thérapeutique étant comprise entre 0,5 et 5,0 µg/mm² de surface et étant appliquée uniquement sur le côté abluminal du système de stent à élution de médicament.

2. Le système de stent à élution de médicament selon la revendication 1, dans lequel le au moins un agent thérapeutique est un composé immunosuppresseur trièno-macrocyclique.

3. Le système de stent à élution de médicament selon la revendication 1, dans lequel le au moins un agent thérapeutique présente la structure suivante : 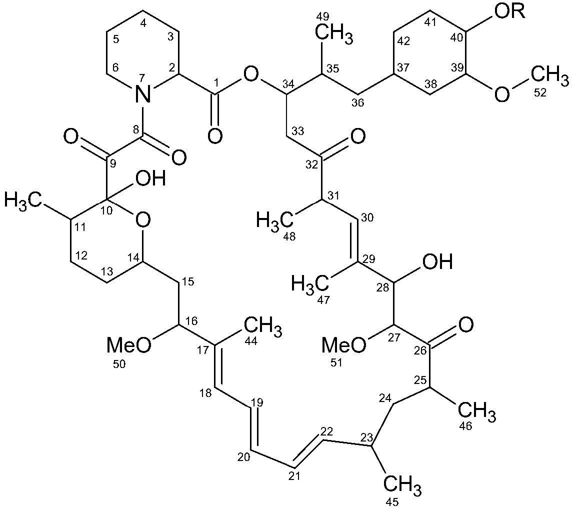 où R est C(O)-(CH₂)n-X, n est 0, 1 ou 2, X est un hydrocarbure cyclique comportant 3 à 9 atomes de carbone et contenant éventuellement une ou plusieurs liaisons insaturées.

4. Le système de stent à élution de médicament selon la revendication 3, dans lequel, en outre, C(O)-(CH₂)n-X présente l'une des structures suivantes :

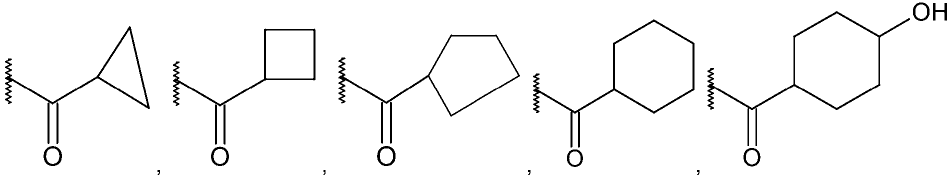

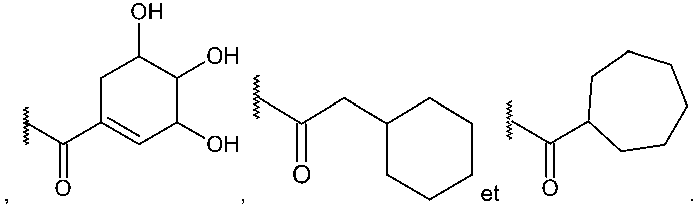

5. Le système de stent à élution de médicament selon la revendication 1, dans lequel le recouvrement de surface poreux est un tissu non tissé.

6. Le système de stent à élution de médicament selon la revendication 5, dans lequel le tissu non tissé est composé de fibres électrofilées.

7. Le système de stent à élution de médicament selon la revendication 6, dans lequel les fibres électrofilées sont constituées d'un polymère sélectionné dans le groupe comprenant ou consistant en du polytétrafluoroéthylène, du fluorure d'éthylène-propylène, du Dacron, du polyéthylène téréphtalate, des polyuréthanes, du polycarbonate, du polypropylène, du Pebax, du polyéthylène et des polymères biologiques tels que le collagène, la fibrine et l'élastine.

8. Le système de stent à élution de médicament selon la revendication 1, dans lequel le recouvrement de surface poreux présente un gradient de porosité allant de 0 % de porosité à la surface luminale à 20 à 40 % de porosité à la surface abluminale.

9. Le système de stent à élution de médicament selon la revendication 1, dans lequel la quantité du au moins un agent thérapeutique est comprise entre 4,0 µg/mm² et 5,0 µg/mm² de surface et est appliquée uniquement sur le côté abluminal du système de stent à élution de médicament.

10. Procédé de préparation d'un système de stent à élution de médicament, comprenant les étapes consistant à:
- (a) fournir deux feuilles différentes d'un matériau poreux,
- (b) monter l'une des deux feuilles sur le côté luminal du stent, tandis que la seconde feuille de matériau poreux est montée sur le côté abluminal du stent, de sorte que la porosité augmente de la surface luminale à la surface abluminale du système de stent, en ce que la feuille située sur la surface luminale du stent présente une porosité inférieure à celle de la feuille de matériau poreux montée sur la surface abluminale du stent, ou ne présente aucune porosité,
- (c) appliquer une température élevée et une pression pour encapsuler le stent dans le matériau poreux, de sorte à fournir un recouvrement de surface poreux sur le stent,
- (d) appliquer une solution d'un agent thérapeutique sur le revêtement de surface poreux, et
- (e) sécher le revêtement de surface poreux auquel la solution d'agent thérapeutique a été appliquée, où, à l'étape (c), la température est comprise dans la plage de 55 °C à 90 °C et la pression est comprise dans la plage de 0,13 MPa à 1,25 MPa.
